# EUROPÄISCHE PATENTSCHRIFT

(11) **EP 2 213 663 B1**
(45) Veröffentlichungstag und Bekanntmachung des Hinweises auf die Patenterteilung: **28.09.2011**
(21) Anmeldenummer: 10151141.8
(22) Anmeldetag: 20.01.2010
(51) Int. Cl.: C07D 239/30

(54) **Herstellung von halogenierten N-heteroaromatischen Polyhalogenderivaten**
Preparation of halogenated N-heteroaromatic polyhalogen derivatives
Préparation de dérivés de poly-halogènes N-hétéro-aromatiques halogénés

(30) Priorität: 29.01.2009 DE 102009006643
(43) Veröffentlichungstag der Anmeldung: 04.08.2010
(73) Patentinhaber: Saltigo GmbH, 40764 Langenfeld (DE)
(72) Erfinder: Marhold, Albrecht, 51373 Leverkusen (DE); Ebenbeck, Wolfgang, 51373 Leverkusen (DE); Knauer, Stephan, 50823 Köln (DE)
(74) Vertreter: Pettrich, Klaus-Günter

(56) Entgegenhaltungen:
- EP-A- 0 110 690
- EP-A- 0 133 663
- DE-A1- 4 222 518

## Beschreibung

Die vorliegende Erfindung betrifft ein Verfahren zur Herstellung von halogenierten N-heteroaromatischen Polyhalogenderivaten.

Halogenierte N-heteroaromatische Polyhalogenderivate besitzen besondere Bedeutung als Zwischenprodukte für die Synthese von Arzneimitteln, insbesondere von Zytostatika.

Zur Herstellung von halogenierten N-heteroaromatischen Polyhalogenderivaten sind verschiedene Verfahren bekannt.

In D E 4222518 A1 wird die Herstellung von halogenierten N-heteroaromatischen Polyhalogenderivaten, insbesondere des 2,4-Dichlor-5-trifluormethylpyrimidins, ausgehend von 2,4-Dichlor-5-trichlormethylpyrimidin durch Umsetzung mit Antimontrifluorid in Gegenwart von Antimonpentafluorid beschrieben. Nachteilig bei diesem Verfahren sind die geringen Reaktionsausbeuten.

Aus WO 2005/023780 A1 ist eine weitere Synthese zur Herstellung eines halogenierten N-heteroaromatischen Polyhalogenderivates, nämlich des 2,4-Dichlor-5-trifluormethylpyrimidins, durch Umsetzung von 5-Trifluormethyluracil mit Phosphortrichlorid bekannt. Dieses Verfahren weist die Nachteile auf, dass sicherheitstechnisch problematische Edukte verwendet werden und das Verfahren aufgrund der hohen Anzahl an Verfahrensschritten ineffizient ist.

In EP 0133663 ist ein Verfahren zur Fluorierung von Trichlormethylpyrimidinen in Gegenwart von Metallhalogeniden als Katalysatoren offenbart.

In EP 0110690 ist ein Verfahren zur Fluorierung von Trichlormethylpyridinen in Gegenwart von Metallhalogeniden als Katalysatoren beschrieben.

Nachteilig an den vorgenannten Verfahren ist, dass sie entweder ineffizient oder/und sicherheitstechnisch problematisch sind.

Es bestand somit ein Bedürfnis, ein Verfahren bereitzustellen, dass die Nachteile der bisherigen Verfahren nicht aufweist und halogenierte N-heteroaromatische Polyhalogenderivate in guten Ausbeuten effizient in technischem Maßstab liefert.

Überraschenderweise wurde gefunden, dass mit dem erfindungsgemäßen Verfahren die Nachteile des Standes der Technik überwunden werden konnten und halogenierte N-heteroaromatische Polyhalogenderivate in guten Ausbeuten hergestellt werden konnten.

Gegenstand der Erfindung ist daher ein Verfahren zur Herstellung von Verbindungen der Formel (I) 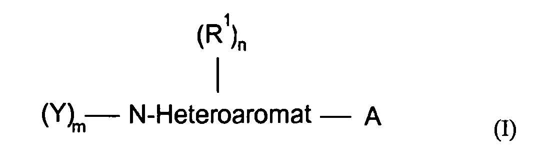 mit A = (C₁-C₆)-Fluoralkyl, (C₁-C₆)-Fluoralkoxy oder (C₁-C₆)-Fluoralkylthio,
N-Heteroaromat = ein 5- gliedriger, aromatischer Heterozyklus mit ein oder zwei Stickstoffatomen oder ein 6- gliedriger, aromatischer Heterozyklus mit zwei oder drei Stickstoffatomen,
R¹ = C₁-C₁₅-Alkyl, C₆-C₁₄-Aryl und/oder C₆-C₁₄-Arylalkyl,
n = 0 oder 1 und
Y = Cl, Br und/oder F ist und Y gleich oder verschieden sein kann und m für 1,2 oder 3 steht, bei dem Verbindungen der Formel (II) 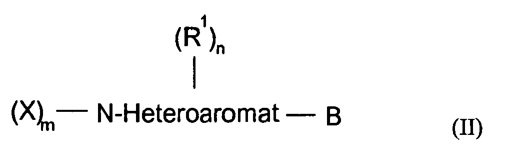 wobei B für ein (C₁-C₆)-Brom- oder -Chloralkyl, (C₁-C₆)-Brom- oder -Chloralkoxy oder (C₁-C₆)-Brom-oder -Chloralkylthio steht und
X = Cl, Br und/oder I ist und X gleich oder verschieden sein kann und
N-Heteroaromat, m, R¹ und n die vorgenannte Bedeutung besitzen,
mit Verbindungen der Formel (III)

Kat¹(F)ᵢ (III)

wobei Kat¹ für ein i-fach geladenes Kation und i für eine natürliche Zahl größer 0 steht,
zu Verbindungen der Formel (IV) umgesetzt werden, 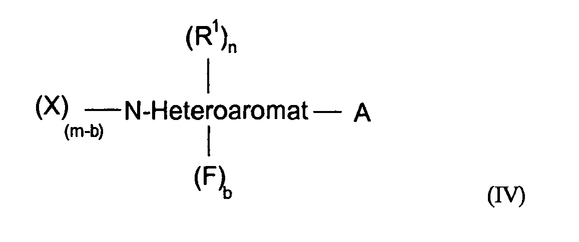 wobei b für 0, 1, 2 oder 3 steht und A, R¹, X, m, n und N-Heteroaromat die vorgenannte Bedeutung besitzen und (m-b) ≥ 0 ist und X ≠ Y für b = 0 ist
und anschließend Verbindungen der Formel (IV) mit Verbindungen der Formel (V)

Kat²(Cl)_{z} oder Kat²(Br)_{z} (V)

wobei Kat² für ein z-fach geladenes Kation und z für eine natürliche Zahl größer 0 steht, durch teilweisen oder vollständigen Halogenaustausch am heteroaromatischen Ring zu Verbindungen der Formel (I) umgesetzt werden und das Verfahren in Abwesenheit einer Lewis-Säure als Katalysator durchgeführt wird.

Der Rahmen der Erfindung umfasst alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Kombination.

N-Heteroaromat steht im Rahmen der Erfindung für einen 5- gliedrigen, aromatischen Heterozyklus mit ein oder zwei Stickstoffatomen oder einen 6- gliedrigen, aromatischen Heterozyklus mit zwei oder drei Stickstoffatomen. Besonders bevorzugt sind zwei Stickstoffatome. Beispielhaft und vorzugsweise seien genannt: Pyridazinyl, Pyrimidinyl, Pyrazinyl, 1,2,3-Triazinyl, 1,2,4-Triazinyl, Pyrrolyl, Pyrazolyl, Imidazolyl und 1,3,5-Triazinyl. Besonders bevorzugt sind Pyridazinyl, Pyrimidinyl und Pyrazinyl. Ganz besonders bevorzugt ist Pyrimidinyl.

Alkyl steht im Rahmen der Erfindung bevorzugt für einen geradkettigen, zyklischen oder verzweigten Alkyl- Rest mit 1 bis 15, besonders bevorzugt 1 bis 6 Kohlenstoffatomen.

Beispielhaft und vorzugsweise steht Alkyl für Methyl, Ethyl, n-Propyl, Isopropyl, n-, i-, (sek.) s-oder (tert.) t-Butyl, Cyclopropyl, Cyclobutyl, Cyclopentyl, Cyclohexyl, Cycloheptyl, n-Pentyl, 1-Methylbutyl, 2-Methylbutyl, 3-Methylbutyl, neo-Pentyl, 1-Ethylpropyl, n-Hexyl, n-Heptyl, n-Octyl, n-Decyl und n-Dodecyl.

Aryl steht im Rahmen der Erfindung für einen mono-, bi- oder trizyklischen carbozyklischen aromatischen Rest mit vorzugsweise 6 bis 14 aromatischen Kohlenstoffatomen (C₆-C₁₄-Aryl). Beispielhaft und bevorzugt steht C₆-C₁₄-Aryl für Biphenyl, Phenyl, Naphthyl, Phenanthrenyl, Anthracenyl oder Fluorenyl.

Arylalkyl bedeutet jeweils unabhängig voneinander einen geradkettigen, zyklischen oder verzweigten Alkyl-Rest nach vorstehender Definition, der einfach, mehrfach oder vollständig durch Aryl-Reste gemäß vorstehender Definition substituiert sein kann. Beispielsweise und vorzugsweise ist Arylalkyl ist Benzyl.

A steht für (C₁-C₆)-Fluoralkyl, (C₁-C₆)- Fluoralkoxy oder (C₁-C₆)-Fluoralkylthio. Bevorzugt ist A ausgewählt aus der Gruppe (C₁-C₄)-Fluoralkyl, (C₁-C₄)-Fluoralkoxy oder (C₁-C₄)-Fluroalkylthio. Besonders bevorzugt ist A (C₁-C₄)-Fluoralkyl.

Fluoralkyl, Fluoralkoxy bzw. Fluoralkylthio stehen im Rahmen der Erfindung für einen geradkettigen, zyklischen oder verzweigten Fluroalkyl-, bzw. Fluoralkoxy-, bzw. Fluoralkylthio-Rest mit 1 bis 6 (C₁-C₆), bevorzugt 1 bis 5 (C₁-C₅) und besonders bevorzugt 1 bis 4 (C₁-C₄)-Kohlenstoffatomen, der teilweise oder vollständig mit Fluoratomen substituiert ist.

Beispielsweise steht Fluoralkyl für Difluorethyl, Difluormethyl, Fluormethyl, Fluorethyl, Fluorpropyl, Trifluorethyl, Trifluormethyl, Tetrafluorethyl, Pentafluorethyl, Heptafluorpropyl, cyclo-Nonafluorpentyl, Heptafluorisopropyl, Hexafluorisopropyl und/oder Nonafluorbutyl. Bevorzugt steht Fluoralkyl für Difluormethyl, Trifluormethyl, Fluorpropyl, Trifluorethyl und/oder Pentafluorethyl.

Beispielsweise steht Fluoralkoxy für Difluorethoxy, Difluormethoxy, Fluormethoxy, Fluorethoxy, Fluorpropoxy, Trifluorethoxy, Trifluormethoxy, Tetrafluorethoxy, Pentafluorethoxy, Heptafluorpropoxy, cyclo-Nonafluorpentoxy, Heptafluorisopropoxy, Hexafluorisopropoxy und/oder Nonafluorbutoxy. Bevorzugt steht Fluoralkoxy für Difluormethoxy, Trifluormethoxy, Fluorpropoxy, Trifluorethoxy und/oder Pentafluorethoxy.

Beispielhaft steht Fluoralkylthio für Difluorethylthio, Difluormethylthio, Fluormethylthio, Fluorethylthio, Fluorpropylthio, Trifluorethylthio, Trifluormethylthio, Tetrafluorethylthio, Pentafluorethylthio, Heptafluorpropylthio, cyclo-Nonafluorpentylthio, Heptafluorisopropylthio Hexafluorisopropylthio und/oder Nonafluorbutylthio. Bevorzugt steht Fluoralkylthio für Difluormethylthio, Trifluormethylthio, Fluorpropylthio, Trifluorethylthio und/oder Pentafluorethylthio.

B steht für Brom- oder Chloralkyl bzw. Brom- oder Chloralkoxy bzw. Brom- oder Chloralkylthio, die einen geradkettigen, zyklischen oder verzweigten Alkyl, bzw. Alkoxy-, bzw. Alkylthio-Rest mit 1 bis 6 (C₁-C₆), bevorzugt 1 bis 5 (C₁-C₅) und besonders bevorzugt 1 bis 4 (C₁-C₄)-Kohlenstoffatomen tragen. Dieser Rest ist teilweise oder vollständig mit Brom- oder Chloratomen substituiert.

Beispielhaft steht Brom- oder Chloralkyl für Brommethyl, Bromethyl, Brombutyl, Dibrommethyl, Bromdichlormethyl, Brompropyl, Chlorethyl, Chlorpropyl, Chlorbutyl, Chlorhexyl, Chlormethyl, Chlorbromethyl, Dichlormethyl, Dibromchlormethyl, Dichlorethyl, Dichlorbromethyl, cyclo-Nonachlorpentyl, Heptachlorpropyl, Heptachlorisopropyl, Hexachlorisopropyl, Hexabromisopropyl, Trichlormethyl, Pentachlorethyl und/oder Nonachlorbutyl. Bevorzugt steht Brom- oder Chloralkyl für Dichlormethyl, Trichlormethyl, Pentachlorethyl, Heptachlorpropyl und/oder Heptachlorisopropyl.

Beispielhaft steht Brom- oder Chloralkoxy für Brommethoxy, Bromethoxy, Brombutoxy, Dibrommethoxy, Bromdichlormethoxy, Brompropoxy, Chlorethoxy, Chlorpropoxy, Dibromchlormethoxy, Dichlormethoxy, Dichlorethoxy, Chlorbutoxy, Chlorhexoxy, Chlormethoxy, Dichlorbromethoxy, Trichlormethoxy, Pentachlorethoxy, Heptachlorpropoxy, Chlorbromethoxy, cyclo-Nonachlorpentoxy, Heptachlorisopropoxy, Hexachlorisopropoxy, Hexabromisopropoxy und/oder Nonachlorbutoxy. Bevorzugt steht Brom- oder Chloralkoxy für Dichlormethoxy, Trichlormethoxy, Pentachlorethoxy, Heptachlorpropoxy und/oder Hexachlorisopropyl.

Beispielhaft steht Brom- oder Chloralkylthio für Brommethylthio, Bromethylthio, Brombutylthio, Brompropylthio, Dibrommethylthio, Bromdichlormethylthio, Chlorethylthio, Chlorpropylthio, Dibromchlormethylthio, Dichlorbromethylthio, Dichlormethylthio, Dichlorethylthio, Chlorbutylthio, Chlorhexylthio, Chlormethylthio, Trichlormethylthio, Tetrachlorbromethylthio, Pentachlorethylthio, Heptachlorpropylthio, cyclo-Nonachlorpentylthio, Heptachlorisopropylthio, Hexachlorisopropylthio, Hexabromisopropylthio und/oder Nonachlorbutylthio. Bevorzugt steht Brom- oder Chloralkylthio für Dichlormethylthio, Trichlormethylthio, Pentachlorethylthio, Heptachlorpropylthio und/oder Hexachlorisopropylthio.

R¹ stellt bevorzugt einen (C₁-C₆)-Alkylrest oder einen gegebenenfalls substituierten Phenyl- oder Benzylrest dar.
Kat¹ und Kat² können gleich oder verschieden sein und stellen unabhängig voneinander Wasserstoffkationen, Alkali- oder Erdalkalimetallkationen, Metallkationen, organische Kationen oder Gemische dieser Kationen dar. Bevorzugt stellen Kat¹ oder/und Kat² Alkalimetallkationen und/oder Wasserstoffkationen dar. Ganz besonders bevorzugt stehen Kat¹ und/oder Kat² für Wasserstoffkationen.

X ist vorzugsweise Chlor oder Brom, besonders bevorzugt Chlor. In dem Fall, dass X in den Verbindungen der Formel (II) Iod darstellt, wird dieses Iod im erfindungsgemäßen Verfahren vollständig durch ein anderes Halogenatom ersetzt.

Y ist vorzugsweise Chlor oder Fluor, besonders bevorzugt ist Y = Chlor.

b ist vorzugsweise eine Zahl 1 oder 2, besonders bevorzugt ist b = 2.

n ist bevorzugt 0 oder 1, besonders bevorzugt ist n = 0.

m ist bevorzugt 1 oder 2, besonders bevorzugt ist m = 2.

Bevorzugt ist (m - b) = 0.

Bevorzugt werden die Verbindungen der Formel (I) aus folgenden Verbindungen der Formel (II) hergestellt:

| Verbindung der Formel (I) | Verbindungen der Formel (II) |
|---|---|
| 2,4-Dichlor-5-trifluormethylpyrimidin | 2,4-Dichlor-5-trichlormethylpyrimidin |
| 2,4-Dichlor-6-trifluormethylpyrimidin | 2,4-Dichlor-6-trichlormethylpyrimidin |
| 4,5-Dichlor-6-trifluormethylpyrimidin | 4,5-Dichlor-6-trichlormethylpyrimidin |
| 3,4-Dichlor-5-trifluormethylpyridazin | 3,4-Dichlor-5-trichlormethylpyridazin |
| 3,6-Dichlor-4-trifluormethylpyridazin | 3,6-Dichlor-4-trichlormethylpyridazin |
| 2,3-Dichlor-5-trifluormethylpyrazin | 2,3-Dichlor-5-trichlormethylpyrazin |
| 2,6-Dichlor-3-trifluormethylpyrazin | 2,6-Dichlor-3-trichlormethylpyrazin |

Besonders bevorzugt wird die Verbindung der Formel (I) 2,4-Dichlor-5-trifluormethylpyrimidin, aus der Verbindung der Formel (II) 2,4-Dichlor-5-trichlormethylpyrimidin hergestellt.

Verbindungen der Formel (III) im Sinne der Erfindung sind Fluorierungsmittel und sind ausgewählt aus der Gruppe HF, Salze von HF, wie z.B. Alkali- oder Erdalkalimetallfluoride, wie z.B. KF, NaF, oder Metallfluoride, wie z.B. SbF₃, oder organische Ammoniumfluoride, wie z.B. Et₃N x 3HF oder Pyridin x 9HF. Bevorzugt sind die Verbindungen der Formel (III) HF oder Gemische aus HF und/oder deren Salzen.

Verbindungen der Formel (V) im Sinne der Erfindung sind Halogenierungsmittel , wie z.B. HCl, HBr, Salze der Halogenwasserstoffsäuren HCl oder HBr, wie z.B. Alkali- oder Erdalkalimetallhalogenide oder Metallhalogenide, wie z.B. AlCl₃, TiCl₄, NaCl, NaBr, KBr, KCI, LiCl, FeCl₃ und/oder CaCl₂. Es können z.B. auch Mischungen aus Salzen gleicher Halogenide und /oder gleicher Halogenwasserstoffsäuren eingesetzt werden.
Bevorzugte Verbindungen der Formel (V) sind HCl und/oder deren Salze, wie Alkali- oder Erdalkalimetallchloride oder Metallchloride. Besonders bevorzugte Verbindung der Formel (V) sind HCl und Gemische aus HCl und/oder deren Salze.

Viele der Verbindungen der Formel (II) und der Verbindungen der Formel (III) und der Verbindungen der Formel (V) sind handelsüblich. Die Herstellung der Verbindungen der Formel (II) und der Verbindungen der Formel (III) und der Verbindungen der Formel (V) kann aber auch nach analogen, aus dem Stand der Technik bekannten Verfahren erfolgen und ist dem Fachmann bekannt.

Die Herstellung der Verbindungen der Formel (II) kann aber auch beispielsweise nach dem in Journal of Fluorine Chemistry, 77, 1996, S. 93-95, offenbarten Verfahren erfolgen.

Das erfindungsgemäße Verfahren kann in Gegenwart oder in Abwesenheit eines organischen Lösungsmittels durchgeführt werden. Als organische Lösungsmittel können polare aliphatische oder aromatische Kohlenwasserstoffe, wie z.B. Alkanole, wie z.B. Methanol, Ethanol, Propanol, aromatische Alkohole wie Benzylalkohol, Phenylethanol, Phenoxyethanol, Ether, wie z.B. , tert.-Butyl-Methylether, Diethylether, Diisopropylether, Dioxan, Tetrahydrofuran und/oder Ethylenglykoldimethyl- oder -diethylether; Ketone, wie z.B. Aceton, Butanon oder Methyl-isobutylketon; Amide, wie z.B. N,N-Dimethylformamid, N,N-Dimethylacetamid, N-Methylformanilid, N-Methylpyrrolidinon, Sulfolane, wie z.B. Tetramethylensulfon, und/oder Sulfoxide, wie z.B. Dimethylsulfoxid eingesetzt werden. In einer bevorzugten Ausführungsform des erfindungsgemäßen Verfahrens handelt es sich bei den organischen polaren Lösungsmitteln um Sulfoxide, wie z.B. Dimethylsulfoxid, Sulfolane, wie z.B. Tetramethylensulfon oder Amide, wie z.B. N-Methylpyrolidinon. Bevorzugt ist die Verwendung von Lösungsmitteln insbesondere dann, wenn metallhaltige Verbindungen der Formel (III) oder (V) eingesetzt werden. Besonders bevorzugt ist es allerdings, die Reaktion in Abwesenheit eines Lösungsmittels durchzuführen.

Das erfindungsgemäße Verfahren kann beispielsweise in Gegenwart geeigneter Katalysatoren durchgeführt werden. Als Katalysatoren für den Halogenaustausch durch Fluor können z.B. Guanidiniumsalze und Tetraphenylphosphoniumhalogenide oder weitere speziellere Guanidiniumsalze, wie sie insbesondere in EP-A 1 266 904 beschrieben werden, eingesetzt werden.

In einer Ausführungsform des erfindungsgemäßen Verfahrens beträgt das Stoffmengenverhältnis der Verbindungen der Formel (III) und der Verbindungen der Formel (II) pro auszutauschendem Halogenatom zwischen 10:1 und 1:2 mol, bevorzugt zwischen 8:1 und 1:2 mol und besonders bevorzugt zwischen 5:1 und 1:1 mol.

In einer weiteren Ausführungsform der Erfindung liegt das Stoffmengenverhältnis der Verbindungen der Formel (V) und der Verbindungen der Formel (IV) pro auszutauschendem Halogenatom zwischen 10:1 1 und 1:2 mol, bevorzugt zwischen 5:1 und 1:2 mol und besonders bevorzugt zwischen 2:1 und 1:1 mol.

Bei der Reaktion der Verbindungen der Formel (II) mit den Verbindungen der Formel (III) beträgt die Reaktionstemperatur vorzugsweise 20 °C bis 300 °C, besonders bevorzugt 100 °C bis 200 °C, ganz besonders bevorzugt 100 °C bis 160 °C.

Bei der Reaktion der Verbindungen der Formel (IV) mit den Verbindungen der Formel (V) beträgt die Reaktionstemperatur vorzugsweise 20 °C bis 300 °C, besonders bevorzugt 120 °C bis 220 °C, ganz besonders bevorzugt 150 °C bis 210 °C.

Bei der Reaktion der Verbindungen der Formel (II) mit den Verbindungen der Formel (III) beträgt der Druck vorzugsweise 1 bis 200 bar, besonders bevorzugt 1 bis 100 bar, ganz besonders bevorzugt 1 bis 25 bar.

Bei der Reaktion der Verbindungen der Formel (IV) mit den Verbindungen der Formel (V) beträgt der Druck vorzugsweise 1 bis 200 bar, besonders bevorzugt 5 bis 100 bar, ganz besonders bevorzugt 10 bis 60 bar.

Die Reaktionszeit der Verbindungen der Formel (II) mit den Verbindungen der Formel (III) beträgt in der Regel 2 bis 20 Stunden, besonders bevorzugt 3 bis 10 Stunden, ganz besonders bevorzugt 3 bis 8 Stunden.

Die Reaktionszeit der Verbindungen der Formel (IV) mit den Verbindungen der Formel (V) beträgt vorzugsweise 1 bis 30 Stunden, besonders bevorzugt 1 bis 20 Stunden, ganz besonders bevorzugt 1 bis 12 Stunden.

Die Reaktion kann beispielsweise so durchgeführt werden, dass zunächst die Verbindungen der Formel (II) vorgelegt werden und dann die Verbindungen der Formel (III) hinzugegeben werden. Ebenso können aber auch zunächst die Verbindungen der Formel (III) vorgelegt werden und dann die Verbindungen der Formel (II) hinzugeben werden. Dann kann z.B. ein inertes Gas, wie z.B. Stickstoff oder Argon, aufgedrückt werden. Beispielsweise können die Verbindungen der Formel (II) dann mit den Verbindungen der Formel (III) unter Druck, z.B. bis zur Druckkonstanz oder/und bis zum Ende der Reaktionszeit, umgesetzt werden. Danach kann beispielsweise die überschüssige Verbindungen der Formel (III), z.B. durch Destillation, entfernt werden und dann die Verbindungen der Formel (V) hinzugegeben werden. Es können aber ebenso die Verbindungen der Formel (V) ohne Abtrennung überschüssiger Verbindungen der Formel (III) hinzugegeben werden. Danach können die Verbindungen der Formel (IV) mit den Verbindungen der Formel (V), z.B. bis zur Druckkonstanz oder/und bis zum Ende der Reaktionszeit, umgesetzt werden. Bevorzugt werden die überschüssigen Verbindungen der Formel (III) nach Beendigung der Reaktion mit den Verbindungen der Formel (II) abgetrennt, vorzugsweise durch Destillation, bevor die Verbindungen der Formel (V) hinzugegeben werden.

Beispielsweise können die Verbindungen der Formel (IV) vor der weiteren Umsetzung mit den Verbindungen der Formel (V) abgetrennt und aufgereinigt werden. Die Verbindungen der Formel (IV) können aber z.B. auch ohne weitere Aufreinigung zu Verbindungen der Formel (I) umgesetzt werden. Bevorzugt werden die Verbindungen der Formel (IV) ohne weitere Aufreinigung mit den Verbindungen der Formel (V) umgesetzt.

Vorzugsweise wird die Reaktion im Wesentlichen wasserfrei durchgeführt. Im Wesentlichen wasserfrei bedeutet, dass der Wassergehalt, bezogen auf die Menge der eingesetzten Reaktionsmischung vorzugsweise zwischen 0,0001 Gew.% und 0,1 Gew.% liegt.

In einer Ausführungsform der Erfindung wird die Reaktion mit den Verbindungen der Formel (III) und den Verbindungen der Formel (II) bis zur Druckkonstanz bzw. bis zum vollständigen Halogenaustausch, durchgeführt. In einer weiteren Ausführungsform der Erfindung wird die Reaktion mit den Verbindungen der Formel (IV) und den Verbindungen der Formel (V) bis zur Druckkonstanz bzw. bis zum vollständigen Halogenaustausch am heteroaromatischen Ring durchgeführt.

Die Reaktion der Verbindungen der Formel (II) mit den Verbindungen der Formel (III) und/oder die Reaktion der Verbindungen der Formel (IV) mit den Verbindungen der Formel (V) kann allerdings auch vorzeitig abgebrochen werden, so dass nicht alle in den Verbindungen der Formel (II) oder/und (IV) vorhandenen Halogene am heteroaromatischen Ring durch andere Halogene ersetzt werden. Bevorzugt wird das erfindungsgemäße Verfahren mit den Verbindungen der Formel (III) bis zur Druckkonstanz bzw. bis zum vollständigen Halogenaustausch durchgeführt. Bevorzugt wird das erfindungsgemäße Verfahren mit den Verbindungen der Formel (V) bis zur Druckkonstanz bzw. bis zum vollständigen Austausch der Halogene am heteroaromatischen Ring durchgeführt.

Bevorzugt wird die Reaktion in einem Autoklaven durchgeführt. Des Weiteren wird bevorzugt unter inerten Bedingungen gearbeitet. Die Inertisierung kann z.B. durch kontinuierliche oder diskontinuierliche Durchströmung der Reaktionsmischung mit inerten Gasen, wie z.B. Argon und Stickstoff, durchgeführt werden.

In einer besonders bevorzugten Ausführungsform wird die Verbindung der Formel (II) mit der Verbindung der Formel (III) in Kontakt gebracht. Es wird Stickstoff aufgedrückt und die Reaktionsmischung erhitzt. Nach Beendigung der Reaktion wird überschüssige Verbindung der Formel (III) abgetrennt, vorzugsweise abdestilliert. Danach wird eine Verbindung der Formel (V) zugegeben, erwärmt und die Reaktion bis zur Druckkonstanz bzw. bis zum vollständigen Austausch der Halogene am heteroaromatischen Ring durchgeführt.

Die erfindungsgemäß hergestellten Verbindungen der Formel (I) eignen sich insbesondere als Zwischenprodukte z.B. zur Herstellung von Arzneimitteln.

Auf erfindungsgemäße Weise können die Verbindungen der Formel (I) in guten Ausbeuten in technischen Prozessen hergestellt werden. Die Aufarbeitung kann in an sich bekannter Weise, z.B. durch Extraktion mit bekannten Lösungsmitteln, wie beispielsweise Essigester, erfolgen.

Das nachstehende Beispiel dient der Erläuterung der Erfindung.

### Beispiele

### 1. Herstellung von 2,4-Dichlor-5-trifluormethylpyrimidin

133.2 g (0.5 mol) 2,4-Dichlor-5-trichlorpyrimidin werden in einem Autoklaven vorgelegt und auf 0 °C heruntergekühlt. 170.7g (8.5 mol) wasserfreie HF werden langsam zudosiert und 10 bar Stickstoff aufgedrückt. Die Reaktionsmischung wird auf 150 °C erwärmt und die Reaktion weitere 4 h bei einem Druck von 21 bar und einer Temperatur von 150 °C fortgeführt. Danach wird die Reaktionsmischung auf 10 °C abgekühlt und überschüssiges HF unter vermindertem Druck abdestilliert. Dann wird 35 bar HCl aufgedrückt und die Reaktion auf 165 °C erwärmt. Die Reaktionsmischung wird bis zur Druckkonstanz, mindestens jedoch 3 h, gerührt. Die Destillation des Rohproduktes bei vermindertem Druck ergibt 74.1g (0.34 mol, 68 %) 2,4-Dichlor-5-trifluormethylpyrimidin.

## Patentansprüche

1. Verfahren zur Herstellung von Verbindungen der Formel (I) 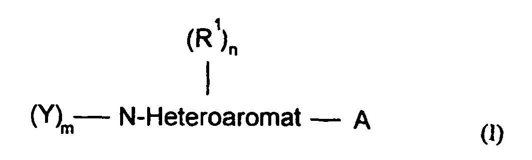 mit A = (C₁-C₆)-Fluoralkyl, (C₁-C₆)-Fluoralkoxy oder (C₁-C₆)-Fluoralkylthio,
N-Heteroaromat = ein 5- gliedriger, aromatischer Heterozyklus mit ein oder zwei Stickstoffatomen oder ein 6- gliedriger, aromatischer Heterozyklus mit zwei oder drei Stickstoffatomen,
R¹ = C₁-C₁₅-Alkyl, C₆-C₁₄-Aryl und/oder C₆-C₁₄-Arylalkyl,
n = 0 oder 1 und
Y = Cl, Br und/oder F ist und Y gleich oder verschieden sein kann und m für 1, 2 oder 3 steht,
**dadurch gekennzeichnet, dass** Verbindungen der Formel (II) 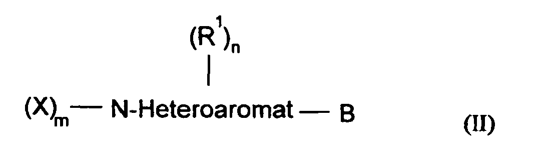 wobei B für ein (C₁-C₆)-Brom- oder -Chloralkyl, (C₁-C₆)-Brom- oder -Chloralkoxy oder (C₁-C₆)-Brom- oder -Chloralkylthio steht und
X = Cl, Br und/oder I ist und X gleich oder verschieden sein kann und
N-Heteroaromat, m, R¹ und n die vorgenannte Bedeutung besitzen,
mit Verbindungen der Formel (III)
Kat¹(F)ᵢ (III)
wobei Kat¹ für ein i-fach geladenes Kation und i für eine natürliche Zahl größer 0 steht,
zu Verbindungen der Formel (IV) umgesetzt werden, 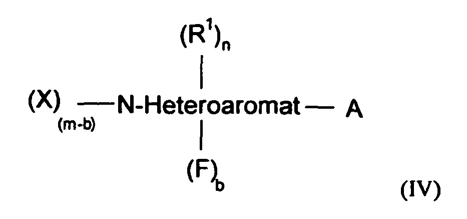 wobei b für 0, 1, 2 oder 3 steht und A, R¹, X, m, n und N-Heteroaromat die vorgenannte Bedeutung besitzen und (m-b) ≥ 0 ist und X ≠ Y für b = 0 ist
und anschließend Verbindungen der Formel (IV) mit Verbindungen der Formel (V)
Kat²(Cl)_{z} oder Kat²(Br)_{z} (V)
wobei Kat² für ein z-fach geladenes Kation und z für eine natürliche Zahl größer 0 steht,
durch teilweisen oder vollständigen Halogenaustausch am heteroaromatischen Ring zu Verbindungen der Formel (I) umgesetzt werden und das Verfahren in Abwesenheit einer Lewis-Säure als Katalysator durchgeführt wird.

2. Verfahren nach Anspruch 1, **dadurch gekennzeichnet, dass** N-Heteroaromat für Pyridazinyl, Pyrimidinyl oder Pyrazinyl steht.

3. Verfahren nach einem der Ansprüche 1 oder 2, **dadurch gekennzeichnet, dass** A in Verbindungen der Formel (I) für Difluormethyl, Trifluormethyl, Trifluorethyl, Pentafluorethyl und Heptafluorisopropyl steht.

4. Verfahren nach einem oder mehreren der Ansprüche 1 bis 3, **dadurch gekennzeichnet, dass** B in Verbindungen der Formel (II) für Dichlormethyl, Trichlormethyl, Trichlorethyl, Pentachlorethyl und Heptachlorisopropyl steht.

5. Verfahren nach einem oder mehreren der Ansprüche 1 bis 4, **dadurch gekennzeichnet, dass** X in Verbindungen der Formel (II) für Chlor steht.

6. Verfahren nach einem oder mehreren der Ansprüche 1 bis 5, **dadurch gekennzeichnet, dass** m = 1 oder 2 ist.

7. Verfahren nach einem oder mehreren der Ansprüche 1 bis 6, **dadurch gekennzeichnet, dass** folgende Verbindungen der Formel (I) aus folgenden Verbindungen der Formel (II) hergestellt werden:
| Verbindung der Formel (I) | Verbindungen der Formel (II) |
|---|---|
| 2,4-Dichlor-5-trifluormethylpyrimidin | 2,4-Dichlor-5-trichlormethylpyrimidin |
| 2,4-Dichlor-6-trifluorinethylpyrimidin | 2,4-Dichlor-6-trichlormethylpyrimidin |
| 4,5-Dichlor-6-trifluormethylpyrimidin | 4,5-Dichlor-6-trichlormethylpyrimidin |
| 3,4-Dichlor-5-trifluormethylpyridazin | 3,4-Dichlor-5-trichlormethylpyridazin |
| 3,6-Dichlor-4-trifluonmethylpyridazin | 3,6-Dichlor-4.trichlormethylpyridazin |
| 2,3-Dichlor-5-trifluormethylpyrazin | 2,3-Dichlor-5-trichlormethylpyrazin |
| 2,6-Dichlor-3-trifluormethylpyrazin | 2,6-Dichlor-3-trichlormethylpyrazin |

8. Verfahren nach einem oder mehreren der Ansprüche 1 bis 7, **dadurch gekennzeichnet, dass** die Verbindung der Formel (III) Fluorwasserstoffsäure ist.

9. Verfahren nach einem oder mehreren der Ansprüche 1 bis 8, **dadurch gekennzeichnet, dass** die Verbindungen der Formel (V) Alkali- oder Erdalkalimetallchloride, Metallchloride oder Chlorwasserstoffsäure sind.

10. Verfahren nach einem oder mehreren der Ansprüche 1 bis 9, **dadurch gekennzeichnet, dass** Verbindungen der Formel (IV) ohne weitere Aufreinigung zu Verbindungen der Formel (I) umgesetzt werden.

11. Verfahren nach einem oder mehreren der Ansprüche 1 bis 10, **dadurch gekennzeichnet, dass** die Reaktion der Verbindungen der Formel (IV) zu den Verbindungen der Formel (I) in Gegenwart eines polaren Lösungsmittels, ausgewählt aus der Gruppe Ether, Ketone, Sulfoxide, Sulfolane oder Amide, durchgeführt wird.

12. Verfahren nach einem oder mehreren der Ansprüche 1 bis 11, **dadurch gekennzeichnet, dass** überschüssige Verbindungen der Formel (III) während der Reaktion aus dem Reaktionsgemisch entfernt werden.

13. Verfahren nach einem oder mehreren der Ansprüche 1 bis 12, **dadurch gekennzeichnet, dass** die Reaktion der Verbindungen der Formel (II) mit den Verbindungen der Formel (III) bei Temperaturen von 100 bis 160 °C durchgeführt wird.

14. Verfahren nach einem oder mehreren der Ansprüche 1 bis 13, **dadurch gekennzeichnet, dass** die Reaktion der Verbindungen der Formel (IV) mit den Verbindungen der Formel (V) bei Temperaturen von 150 bis 210 °C durchgeführt wird.

## Claims

1. Process for the preparation of compounds of the formula (I) 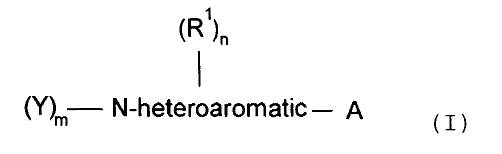 where A = (C₁-C₆)-fluoroalkyl, (C₁-C₆)-fluoroalkoxy or (C₁-C₆)-fluoroalkylthio,
N-heteroaromatic = a 5-membered, aromatic heterocycle with one or two nitrogen atoms or a 6-membered, aromatic heterocycle with two or three nitrogen atoms, R¹ = C₁-C₁₅-alkyl, C₆-C₁₄-aryl and/or C₆-C₁₉-arylalkyl,
n = 0 or 1 and
Y = Cl, Br and/or F and Y may be identical or different and m is 1, 2 or 3,
**characterized in that** compounds of the formula (II) 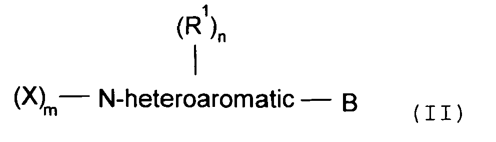 where B is a (C₁-C₆)-bromo- or -chloroalkyl, (C₁-C₆)-bromo- or -chloroalkoxy or (C₁-C₆)-bromo- or - chloroalkylthio and
X = Cl, Br and/or I and X may be identical or different and
N-heteroaromatic, m, R¹ and n have the above meaning, P10100046-EP
are reacted with compounds of the formula (III)
Cat¹(F)ᵢ (III)
where Cat¹ is an i-fold charged cation and i is a natural number greater than 0,
to give compounds of the formula (IV), 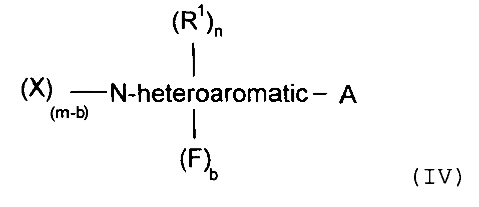 where b is 0, 1, 2 or 3, and A, R¹, X, m, n and N-heteroaromatic have the above meaning and (m-b) ≥ 0 and X ≠ Y when b = 0
and then compounds of the formula (IV) are reacted with compounds of the formula (V)
Cat²(Cl)_{z} or Cat²(Br)_{z} (V)
where Cat² is a z-fold charged cation and z is a natural number greater than 0,
through partial or complete halogen exchange on the heteroaromatic ring to give compounds of the formula (I) and the process is carried out in the absence of a Lewis acid as catalyst.

2. Process according to Claim 1, **characterized in that** N-heteroaromatic is pyridazinyl, pyrimidinyl or pyrazinyl.

3. Process according to one of Claims 1 or 2, **characterized in that** A in compounds of the formula (I) is difluoromethyl, trifluoromethyl, trifluoroethyl, pentafluoroethyl and heptafluoroisopropyl.

4. Process according to one or more of Claims 1 to 3, **characterized in that** B in compounds of the formula (II) is dichloromethyl, trichloromethyl, trichloroethyl, pentachloroethyl and heptachloroisopropyl.

5. Process according to one or more of Claims 1 to 4, **characterized in that** X in compounds of the formula (II) is chlorine.

6. Process according to one or more of Claims 1 to 5, **characterized in that** m = 1 or 2.

7. Process according to one or more of Claims 1 to 6, **characterized in that** the following compounds of the formula (I) are prepared from the following compounds of the formula (II):
| Compound of the formula (I) | Compounds of the formula (II) |
|---|---|
| 2,4-Dichloro-5-trifluoromethylpyrimidine | 2,4-Dichloro-5-trichloromethylpyrimidine |
| 2,4-Dichloro-6-trifluoromethylpyrimidine | 2,4-Dichloro-6-trichloromethylpyrimidine |
| 4,5-Dichloro-6-rifluoromethylpyrimidine | 4,5-Dichloro-6-trichloromethylpyrimidine |
| 3,4-Dichloro-5-trifluoromethylpyridazine | 3,4-Dichloro-5-trichloromethylpyridazine |
| 3,6-Dichloro-4-trifluoromethylpyridazine | 3,6-Dichloro-4-trichloromethylpyridazine |
| 2,3-Dichloro-5-trifluoromethylpyrazine | 2,3-Dichloro-5-trichloromethylpyrazine |
| 2,6-Dichloro-3-trifluoromethylpyrazine | 2,6-Dichloro-3-trichloromethylpyrazine |

8. Process according to one or more of Claims 1 to 7, **characterized in that** the compound of the formula (III) is hydrofluoric acid.
P10100046-EP

9. Process according to one or more of Claims 1 to 8, **characterized in that** the compounds of the formula (V) are alkali metal or alkaline earth metal chlorides, metal chlorides or hydrochloric acid.

10. Process according to one or more of Claims 1 to 9, **characterized in that** compounds of the formula (IV) are reacted without further purification to give compounds of the formula (I).

11. Process according to one or more of Claims 1 to 10, **characterized in that** the reaction of the compounds of the formula (IV) to give the compounds of the formula (I) is carried out in the presence of a polar solvent, selected from the group consisting of ethers, ketones, sulphoxides, sulpholanes or amides.

12. Process according to one or more of Claims 1 to 11, **characterized in that** excess compounds of the formula (III) are removed from the reaction mixture during the reaction.

13. Process according to one or more of Claims 1 to 12, **characterized in that** the reaction of the compounds of the formula (II) with the compounds of the formula (III) is carried out at temperatures of from 100 to 160°C.

14. Process according to one or more of Claims 1 to 13, **characterized in that** the reaction of the compounds of the formula (IV) with the compounds of the formula (V) is carried out at temperatures of from 150 to 210°C.

## Revendications

1. Procédé pour la préparation de composés de formule (I) 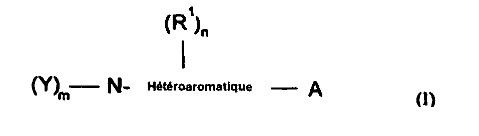 où
A = (C₁-C₆)-fluoroalkyle, (C₁-C₆)-fluoroalcoxy ou (C₁- C₆)-fluoroalkylthio,
N-hétéroaromatique = un hétérocycle aromatique de 5 chaînons, comprenant un ou deux atomes d'azote ou un hétérocycle aromatique de 6 chaînons, comprenant deux ou trois atomes d'azote,
R¹ = C₁-C₁₅-alkyle, C₆-C₁₄-aryle et/ou C₆-C₁₄- arylalkyle,
n = 0 ou 1 et
Y = Cl, Br et/ou F et Y peut être identique ou différent et m vaut 1, 2 ou 3,
**caractérisé en ce qu'**on transforme des composés de formule (II) 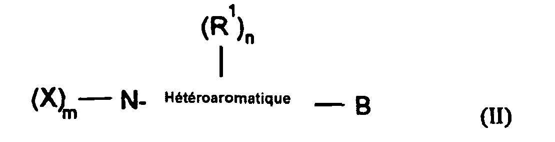 où
B représente un (C₁-C₆)-bromoalkyle ou un (C₁-C₆)- chloroalkyle, un (C₁-C₆)-bromoalcoxy ou un (C₁-C₆)- chloroalcoxy ou un (C₁-C₆)-bromoalkylthio ou un (C₁-C₆)-chloroalkylthio et
X = Cl, Br et/ou I et X peut être identique ou différent et
N-hétéroaromatique, m, R¹ et n ont la signification susmentionnée,
avec des composés de formule (III)
Cat¹(F)ᵢ (III)
où Cat¹ représente un cation à i charges et i représente un nombre naturel supérieur à 0, en composés de formule (IV), 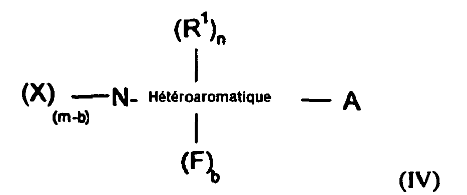 où b vaut 0, 1, 2 ou 3 et A, R¹, X, m, n et N-hétéroaromatique ont la signification susmentionnée et (m-b ) ≥ 0 et X ≠ Y pour b = 0
puis on transforme les composés de formule (IV) avec des composés de formule (V)
Cat²(Cl)_{z} ou Cat²(Br)_{z} (V)
où Cat² représente un cation à z charges et z représente un nombre naturel supérieur à 0,
par échange partiel ou complet des halogènes sur le cycle hétéroaromatique en composés de formule (I) et le procédé est réalisé en l'absence d'un acide de Lewis comme catalyseur.

2. Procédé selon la revendication 1, **caractérisé en ce que** N-hétéroaromatique représente pyridazinyle, pyrimidinyle ou pyrazinyle.

3. Procédé selon l'une quelconque des revendications 1 ou 2, **caractérisé en ce que** A dans les composés de formule (I) représente difluorométhyle, trifluorométhyle, trifluoroéthyle, pentafluoroéthyle et heptafluoroisopropyle.

4. Procédé selon l'une ou plusieurs des revendications 1 à 3, **caractérisé en ce que** B dans les composés de formule (II) représente dichlorométhyle, trichlorométhyle, trichloroéthyle, pentachloroéthyle et heptachloroisopropyle.

5. Procédé selon l'une ou plusieurs des revendications 1 à 4, **caractérisé en ce que** X dans les composés de formule (II) représente chlore.

6. Procédé selon l'une ou plusieurs des revendications 1 à 5, **caractérisé en ce que** m = 1 ou 2.

7. Procédé selon l'une ou plusieurs des revendications 1 à 6, **caractérisé en ce que** les composés suivants de formule (I) sont préparés à partir des composés suivants de formule (II) :
| Composé de formule (I) | Composés de formule (II) |
|---|---|
| 2,4-dichloro-5-trifluorométhylpyrimidine | 2,4-dichloro-5-trichlorométhylpyrimidine |
| 2,4-dichloro-6-trifluorométhylpyrimidine | 2,4-dichloro-6-trichlorométhylpyrimidine |
| 4,5-dichloro-6-trifluorométhylpyrimidine | 4,5-dichloro-6-trichlorométhylpyrimidine |
| 3,4-dichloro-5-trifluorométhylpyridazine | 3,4-dichloro-5-trichlorométhylpyridazine |
| 3,6-dichloro-4-trifluorométhylpyridazine | 3,6-dichloro-4-trichlorométhylpyridazine |
| 2,3-dichloro-5-trifluorométhylpyrazine | 2,3-dichloro-5-trichlorométhylpyrazine |
| 2,6-dichloro-3-trifluorométhylpyrazine | 2,6-dichloro-3-trichlorométhylpyrazine |

8. Procédé selon l'une ou plusieurs des revendications 1 à 7, **caractérisé en ce que** le composé de formule (III) est l'acide fluorhydrique.

9. Procédé selon l'une ou plusieurs des revendications 1 à 8, **caractérisé en ce que** les composés de formule (V) sont des chlorures de métal alcalin ou alcalino-terreux, des chlorure de métal ou l'acide chlorhydrique.

10. Procédé selon l'une ou plusieurs des revendications 1 à 9, **caractérisé en ce que** les composés de formule (IV) sont transformés sans autre purification en composés de formule (I).

11. Procédé selon l'une ou plusieurs des revendications 1 à 10, **caractérisé en ce que** la réaction des composés de formule (IV) en composés de formule (I) est réalisée en présence d'un solvant polaire, choisi dans le groupe des éthers, des cétones, des sulfoxydes, des sulfolanes ou des amides.

12. Procédé selon l'une ou plusieurs des revendications 1 à 11, **caractérisé en ce que** les composés en excès de formule (III) sont éliminés du mélange réactionnel pendant la réaction.

13. Procédé selon l'une ou plusieurs des revendications 1 à 12, **caractérisé en ce que** la réaction des composés de formule (II) avec les composés de formule (III) est réalisée à des températures de 100 à 160°C.

14. Procédé selon l'une ou plusieurs des revendications 1 à 13, **caractérisé en ce que** la réaction des composés de formule (IV) avec les composés de formule (V) est réalisée à des températures de 150 à 210°C.
